(19) Europäisches Patentamt
European Patent Office
Office européen des brevets

(11) Publication number: **0 509 401 A1**

(12) **EUROPEAN PATENT APPLICATION**

(21) Application number: **92106161.0**

(51) Int. Cl.$^5$: **A61K 31/645**

(22) Date of filing: **09.04.92**

(30) Priority: **19.04.91 US 688005**

(43) Date of publication of application: **21.10.92 Bulletin 92/43**

(84) Designated Contracting States: **AT BE CH DE DK ES FR GB GR IT LI LU NL PT SE**

(71) Applicant: **HOECHST-ROUSSEL PHARMACEUTICALS INCORPORATED**
**P.O. Box 2500, Route 202-206**
**Somerville, NJ 08876-1258(US)**

(72) Inventor: **Shutske, Gregory Michael**
**21 Coppermine Village**
**Bridgewater, NJ 08807(US)**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

(54) **Use of 9-amino-1,2,3,4-tetrahydroacridin-1-ol and related compounds for the treatment of aids.**

(57) There is disclosed the use of 9-amino-1,2,3,4-tetrahydroacridin-1-ol and related compounds of the formula below,

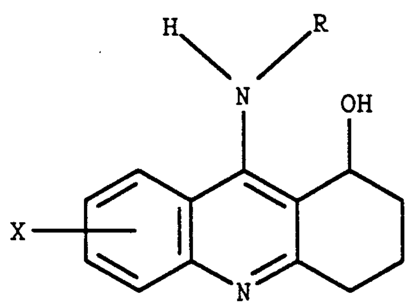

where

X is hydrogen, loweralkyl or halogen; and

R is hydrogen, loweralkyl or benzyl;

for the treatment of acquired immune deficiency syndrome (AIDS).

EP 0 509 401 A1

The present invention relates to the use of 9-amino-1,2,3,4-tetrahydroacridin-1-ol and related compounds having Formula I below,

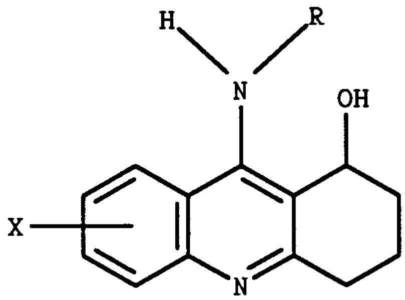

( I )

where

X is hydrogen, loweralkyl or halogen; and

R is hydrogen, loweralkyl or benzyl

for the preparation of a medicament being effective in the treatment of acquired immune deficiency syndrome (AIDS).

Especially preferred compounds for the purpose of the method of this invention are 9-amino-1,2,3,4-tetrahydroacridin-1-ol (Formula II) and 9-benzylamino-1,2,3,4-tetrahydroacridin-1-ol (Formula III).

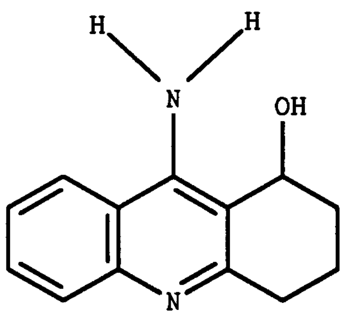

( II )

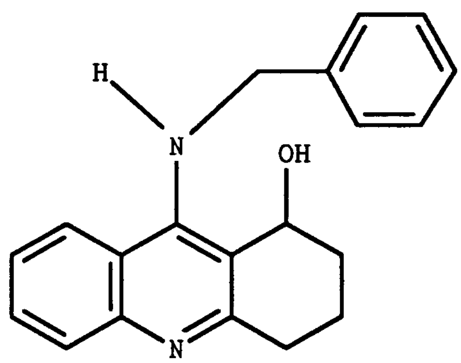

( III )

Throughout the specification and the appended claims, the term loweralkyl shall mean an alkyl group of 1 to 4 carbon atoms; and the term halogen shall mean fluorine, chlorine, bromine or iodine.

Compounds of Formula I are disclosed and claimed in Shutske et al., United States Patent No. 4,631,286, which issued on December 23, 1986. In said Shutske patent, these compounds are disclosed as being useful for the treatment of memory dysfunctions characterized by a cholinergic deficit such as Alzheimer's disease (senile dementia of Alzheimer type).

Certain similarities have been pointed out between the neurological symptoms of HIV (human immune deficiency virus) infection and those of Alzheimer's disease. See for instance, *Fredj et al.*, *"Tetrahydroaminoacridine in HIV Infections", International Journal of Clinical Pharmacology, Therapy and Toxicology, No. 27, Vol. 8, pp. 408-410 (1989)*. In the Fredj article, the authors conducted a short term (up to 60 days) clinical study on the use of 9-amino-1,2,3,4-tetrahydroacridine (tacrine, often abbreviated as THA, Formula IV below) for the treatment of HIV infections. They observed a short term therapeutic value of THA in terms of hematology (total lymphocyte count and CD4 cell count) and serology (serum p24 antigen titer).

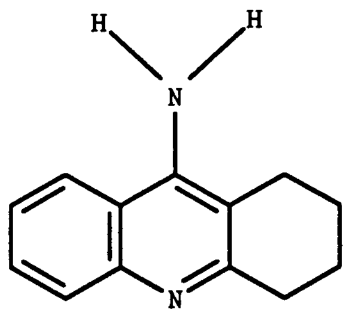

( IV )

According to SCRIP No. 1528, p. 23 (1990), Francois Dietlin et al conducted a comparative trial between tacrine and zidovudine for the treatment of ARC/AIDS patients. In this study, according to the article, 52 patients received tacrine and 59 patients received zidovudine. After a mean treatment duration of 5.4 months in the tacrine group and 5.8 months in the zidovudine group, p24 levels had decreased to a greater extent in the tacrine group than in the ziodovudine group and fewer opportunistic infections had occurred in the former group.

The present inventor believes compounds I are useful for the treatment of HIV infections, although the exact mechanism of action is not known at the present moment. The present inventor believes that the compounds of Formula I have substantial advantages over tacrine in regard to side effects such as acute toxicity. See for instance, Shutske et al., J. Med. Chem., 1989, 32, 1805-1813.

Examples of compounds of Formula I useful for the method of this invention include:
9-amino-1,2,3,4-tetrahydroacridin-1-ol;
9-benzylamino-1,2,3,4-tetrahydroacridin-1-ol;
9-amino-6-methyl-1,2,3,4-tetrahydroacridin-1-ol; and
9-amino-6-chloro-1,2,3,4-tetrahydroacridin-1-ol.

As mentioned earlier, the compound of Formula II (velnacrine) and the compound of Formula III are especially preferred for the purpose of this invention.

Effective quantities of the compounds of the invention may be administered to a patient by any of the various methods, for example, orally as in capsule or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0 - 300 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar,

shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present inventions are prepared so that a parenteral dosage unit contains between 0.5 to 100 milligrams of active compound.

## Claims

1. A medicament for the treatment of acquired immune deficiency syndrome (AIDS), which comprises a compound of the formula I

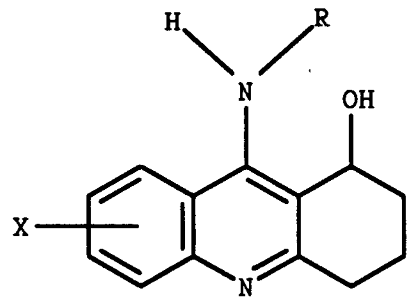

(I)

where

X is hydrogen, loweralkyl or halogen; and

R is hydrogen, loweralkyl or benzyl.

2. The medicament as defined in claim 1 where X and R are hydrogen.

3. The medicament as defined in claim 1 where X is hydrogen and R is benzyl.

4. Use of a compound of the formula I as defined in claim 1 for the preparation of a medicament being effective in the treatment of acquired immune deficiency syndrome (AIDS).

5. The use as defined in claim 4, where in formula I X and R are hydrogen.

6. The use as defined in claim 4, where in formula I X is hydrogen and R is benzyl.

## Claims for the following Contracting State : ES

1. Use of a compound of the formula I

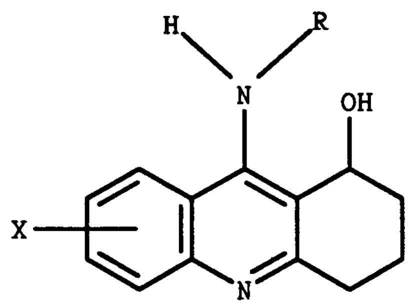

(I)

where
X is hydrogen, loweralkyl or halogen; and
R is hydrogen, loweralkyl or benzyl
for the preparation of a medicament being effective in the treatment of acquired immune deficiency syndrome (AIDS).

**2.** The use as defined in claim 1, wherein X and R are hydrogen.

**3.** The use as defined in claim 1, wherein X is hydrogen and R is benzyl.

European Patent Office

# EUROPEAN SEARCH REPORT

Application Number

EP 92 10 6161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | INTERNATIONAL JOURNAL OF CLINICAL PHARMACOLOGY, THERAPY AND TOXICOLOGY, vol. 27, no. 8, 1989, pages 408-410; G. FREDJ et al.: "Tetrahydroaminoacridine in HIV infections"<br>* Whole document * | 1-6 | A 61 K 31/645 |
| Y | 6TH INTERNATIONAL CONFERENCE AIDS, 20th - 23rd June 1990, abstract no. S.B.457; M. YOULE et al.: "Open study of tacrine hydrochloride in HIV P24 antigen positive patients"<br>* Whole document * | 1-6 | |
| Y | 6TH INTERNATIONAL CONFERENCE AIDS, 20th - 23rd June 1990, abstract no. S.B.458; G. FREDJ et al.: "Follow-up of HIV infected post AZT pateints treated by tacrine (THA)"<br>* Whole document * | 1-6 | |
| D,Y | US-A-4 631 286 (SHUTSKE et al.)<br>* Abstract; claims * | 1-6 | TECHNICAL FIELDS SEARCHED (Int. Cl.5)<br>A 61 K |
| Y | EP-A-0 375 471 (STE CIVILE DE RECHERCHE NEWPHARM)<br>* Whole document * | 1-6 | |

The present search report has been drawn up for all claims

| Place of search | Date of completion of the search | Examiner |
|---|---|---|
| THE HAGUE | 15-06-1992 | GOETZ G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)